# FASCICULE DE BREVET EUROPEEN

(11) **EP 2 306 847 B1**
(45) Date de publication et mention de la délivrance du brevet: **09.11.2016**
(21) Numéro de dépôt: 09737089.4
(22) Date de dépôt: 20.07.2009
(51) Int. Cl.: A23L 29/244, A23L 29/30, A23L 33/10, A23L 33/21, A61K 31/715, A61K 31/721, A61K 35/748, A61K 36/02, C12N 1/16, C08L 5/00, C08L 3/02, A23K 10/18, A23K 20/163, A23K 50/40, A23K 50/00, A61K 31/733, C12N 1/12, A61K 36/06

(54) **COMPOSITION DE MALTODEXTRINES BRANCHEES ET D'ORGANISMES EUCARYOTES DOTES D'UNE PAROI POLYSACCHARIDIQUE UTILISEES DANS LE DOMAINE DU BIEN ETRE.**
ZUSAMMENSETZUNG AUS VERZWEIGTEN MALTODEXTRINEN UND EUKARYONTISCHEN ORGANISMEN MIT POLYSACCHARIDWAND, DIE IM WELLNESS-SEKTOR EINGESETZT WERDEN
COMPOSITION OF BRANCHED MALTODEXTRINS AND EUKARYOTIC ORGANISMS HAVING A POLYSACCHARIDE WALL, USED IN THE FIELD OF WELL-BEING

(30) Priorité: 18.07.2008 FR 0854924
(43) Date de publication de la demande: 13.04.2011
(73) Titulaire: Roquette Frères, 62136 Lestrem (FR)
(72) Inventeur: DEREMAUX, Laëtitia, F-59800 Lille (FR); WILS, Daniel, F-59190 Morbecque (FR)
(74) Mandataire: Cabinet Plasseraud
(86) Numéro de dépôt international: PCT/FR2009/051446
(87) Numéro de publication internationale: WO 2010/007332

(56) Documents cités:
- WO-A1-00/33854
- WO-A1-97/34615
- WO-A1-2006/112364
- WO-A2-2006/134409
- FR-A1- 2 884 422
- US-A1- 2007 009 502
- BOMBA A ET AL: "Improvement of the probiotic effect of micro-organisms by their combination with maltodextrins, fructo-oligosaccharides and polyunsaturated fatty acids" BRITISH JOURNAL OF NUTRITION, 2001 YAKULT INTERNATIONAL CONFERENCE ON PROBIOTICS AND HEALTH; LONDON, ENGLAND; SEPTEMBER 13-14, 2001, vol. 88, no. Supplement 1, 1 septembre 2002 (2002-09-01), pages S95-S99, XP002566319
- HOLZAPFEL W.H.: "Taxonomy and important features of probiotic microorganisms in food and nutrition." AM J CLIN NUTR, [Online] vol. 73, no. suppl., 2001, pages 365S-373S, XP002566513 Extrait de l'Internet: URL:http://www.ajcn.org/cgi/reprint/73/2/3 65S> [extrait le 2010-01-27]
- YOKOSE TAKESHI ET AL: "Growth-Promoting Effect of Alginate Oligosaccharides on a Unicellular Marine Microalga, Nannochloropsis oculata" BIOSCI. BIOTECHNOL. BIOCHEM,, vol. 73, no. 2, 1 février 2009 (2009-02-01), pages 450-453, XP002566327

## Description

La présente invention porte sur l'utilisation en combinaison de maltodextrines branchées et d'au moins un organisme eucaryote doté d'une paroi polysaccharidique dans le domaine du bien être, dans la complémentation alimentaire, dans l'amélioration de la digestion et de la santé des animaux non ruminants.

Du fait de leurs habitudes alimentaires communes, la flore intestinale des animaux non ruminants à savoir des animaux carnivores et omnivores est composée de souches bactériennes de mêmes genres. Au-delà, cette flore évolue en fonction de l'âge et du régime alimentaire de l'hôte. Chez l'homme, le tractus gastrointestinal est constitué d'un écosystème microbien complexe (10¹³ à 10¹⁵ bactéries/g), avec une prédominance des Bactéroïdes, des Bifidobactéries et des Eubactéries. Les microorganismes forment un microbiote qui exerce de nombreuses fonctions biochimiques et physiologiques notamment (i) un complément à la fermentation des nutriments, (ii) un effet de barrière pour protéger le système digestif contre les bactéries pathogènes, (iii) une stimulation dans le développement du système immunitaire.

Ce microbiote, est constitué de plus de 500 espèces différentes connues. Les membres du genre *Bacteroides* représentent de 25 % à 60 % de la population bactérienne dans l'intestin de l'homme adulte (MOORE W-E-C., 1978) (FINEGOLD S-M., 1983).

L'art antérieur décrit que des activités glycanases seraient codées par certaines souches colonisant la muqueuse intestinale.

*Bacteroides thetaiotaomicron* coderait pour 172 glycosylhydrolases, *Bifidobacterium longum* n'en compte que 39. Cependant, *in vitro,* seule l'expression de certaines enzymes a été décrite. En outre, cette expression serait inductible (Salyers AA et al., 1977, Kopecný J, et al., 2004 ; Robert C. et al., 2007).

Ainsi, l'induction de la flore intestinale peut être mise en évidence par la mesure de ces activités glycolytiques telles que notamment des α et β glucosidases, β galactosidase, β galactosidase, cellobiohydrolase et β-xylosidase, (MARTEAU Ph. et al., 1990).

Les maltodextrines branchées selon l'invention sont des fibres indigestibles solubles dans l'eau. Elles sont en effet, non hydrolysables par les enzymes synthétisées par les animaux non ruminants à savoir les carnivores et omnivores et sont fermentescibles autrement dit, elles sont fermentées par la flore bactérienne intestinale de l'hôte, à savoir des animaux carnivores ou omnivores. La fermentation libère des acides gras à courtes chaînes dans le côlon, qui ont pour effet de diminuer le pH du milieu colique et par voie de conséquence, de limiter le développement de bactéries pathogènes.

Les fibres contenues dans les maltodextrines branchées peuvent être dosées selon la méthode AOAC 2001.03.

Les végétaux ont une paroi cellulaire pectocellulosique composée de cellulose, d'hémicelluloses, de xylanes, de mannanes et de protéines, ainsi que de lignine dans de nombreux cas. Les algues et notamment les microalgues eucaryotes qui représentent une classe particulière des végétaux, comportent dans leur paroi, en plus de la cellulose, des copolymères cellulose/mannanes, du chitosane et de la chitine.

La paroi cellulaire des champignons est, elle, composée majoritairement de chitine, alors que la paroi de certains champignons unicellulaires tels que les levures se rapproche de certains végétaux puisqu'elle comporte des glucanes et des mannanes.

Les parois polysaccharidiques des microalgues eucaryotes sont insolubles et sont peu fermentées par la flore intestinale des animaux non-ruminants. Elles sont donc indigestibles par les omnivores et les carnivores tels que les mammifères et notamment les hommes.

Les parois polysaccharidiques des cellules eucaryotes s'opposent à celles des procaryotes à savoir des bactéries ou des cyanobactéries qui elles, sont constituées de peptidoglycanes de structures et de propriétés totalement distinctes.

Parmi les végétaux, on décrit : les algues, les lichens et les plantes supérieures. Par plantes supérieures, on entend « plantes à tige » ou Cormophytes. Les champignons, tels que par exemple les levures, ne sont pas considérés comme des végétaux mais comportent au même titre que ceux-ci une paroi polysaccharidique.

Les végétaux et les champignons unis ou pluricellulaires constituent tout à la fois un potentiel nutritionnel important ainsi qu'une source d'agents antioxydants tels que la lutéine, le sélénium, les caroténoïdes ou la chlorophylle pour les végétaux.

Cependant, du fait de la faible digestibilité de leurs parois polysaccharidiques pour les omnivores, les carnivores et notamment pour l'homme, seule une faible proportion de ces nutriments ou agents antioxydants est libérée dans la lumière de l'intestin.

Ainsi, une partie conséquente des apports nutritifs des végétaux et des champignons n'est pas libérée et donc pas absorbée au cours de la digestion. Ce problème prend une tout autre importance dans le cas d'une alimentation majoritairement voire exclusivement végétale.

Bien que constituant une source de nutriments importante, certains végétaux sont très rarement consommés, tel est le cas des algues. On distingue chez les algues, les macroalgues et les microalgues.

Les macroalgues, qu'elles soient Chlorophytes, Chromophytes ou Rhodophytes, sont connues pour leur richesse en antioxydants et antiradicalaires tels que les caroténoïdes, les polyphénols, les vitamines ou les acides gras polyinsaturés. En ce qui concerne les polyphénols par exemple, certaines macroalgues telles que les algues brunes (Chromophytes) et notamment les fucales comportent des teneurs en polyphénols qui peuvent atteindre 15% de la matière sèche.

Les microalgues et notamment les viridiplantae, les labyrinthulides, les haptophytes, les rhodophytes et les alveolates représentent un large groupe potentiellement fournisseur de composés ayant des activités biologiques valorisables tels que les protéines dont la richesse peut aller jusqu'à 70% en masse sèche de la microalgue. On peut citer aussi les vitamines notamment les vitamines A, B1, B2, B6, B12, C, E, l'acide folique ou l'acide panthoténique, ou les pigments susceptibles d'avoir un effet positif sur la santé tel qu'un effet anti-oxydant pour la chlorophylle, les caroténoïdes ou les phycobiliprotéines.

Les phycobiliprotéines sont des pigments hydrosolubles de la photosynthèse. On les trouve dans les phycobilisomes chez les rhodophycées, ainsi que libres dans le lumen des thylakoïdes chez les Cryptophycées.

On peut citer la phycocyanine dont l'activité antioxydante serait six fois plus importante qu'un composé antioxydant de référence tel que le TROLOX® (l'acide 6-Hydroxy-2,5,7,8-tetramethylchroman-2-carboxylique) et 20 fois supérieure que celle de l'acide ascorbique ou de la vitamine C.

Pour les raisons qui précèdent, les végétaux ainsi que les champignons, sont utilisés comme éléments de choix pour les compléments alimentaires. Cependant, pour que leurs métabolites soient accessibles et assimilables, une extraction par lyse cellulaire est généralement préconisée.

Les techniques classiquement utilisées sont des techniques physiques par ébullition, méthode qui présente l'inconvénient de dégrader les constituants labiles à la chaleur, tels que les vitamines par exemple.

Il existe aussi des techniques chimiques par l'utilisation de solvants tels que le phénol, l'acide formique ou l'urée. L'inconvénient de cette dernière méthode est l'élimination du solvant après la lyse des cellules et la nécessité d'une vérification du caractère non toxique des lysats obtenus. Cette toxicité peut être induite par la présence de composés issus de réactions chimiques non désirées avec le solvant ou induites par le solvant.

Une lyse enzymatique des parois de ces organismes eucaryotes est envisageable mais impose des conditions strictes que ce soit dans la production des enzymes actifs ou dans leur purification suivant le moyen de production choisi et la destination du produit final. Enfin, le choix des enzymes et la détermination des conditions réactionnelles optimales peuvent nécessiter une mise en place laborieuse avant d'obtenir des conditions permettant une reproductibilité de la réaction.

De plus, la lyse et l'élimination des parois cellulaires réduiraient les propriétés de ces cellules eucaryotes dans l'amélioration de la santé et notamment dans la lutte contre les maladies entéropathogéniques.

En effet, certains végétaux ou champignons unicellulaire tels que les levures ou les microalgues, sont décrites comme ayant des propriétés bénéfiques vis-à-vis de la santé intestinale. Ainsi, elles seraient particulièrement conseillées dans le traitement des différents syndromes entériques tels que les gastroentérites infantiles ou les diarrhées... De fait, leur paroi polysaccharidique permettrait l'adsorption de toxines synthétisées par les bactéries entéropathogéniques. Ceci a été décrit pour *Saccharomyces boulardii* et *Saccharomyces cerevisiae* pour les toxines synthétisées par *Vibrio Cholerae, Clostridium difficile ou Escherichia coli entéropathogénique (EPEC).*

En outre, les levures comme les microalgues seraient impliquées dans la protection de la santé en général en adsorbant des xénobiotiques tels que les mycotoxines, les dioxines ou les PCB. Au-delà de la toxicité induite par la perméabilité de la barrière intestinale aux xénobiotiques et à leur passage dans le sang, ces toxines seraient de plus impliquées dans de nombreuses maladies intestinales du fait de l'agression tout à la fois de la muqueuse intestinale et de la flore.

L'ingestion sans lyse préalable des levures ou des algues induit une protection de l'intestin vis-à-vis des bactéries entéropathogéniques et des xénobiotiques. Cependant, en l'absence de lyse de ces organismes le long du tractus, cet effet protecteur pour la santé intestinale est moindre en comparaison au potentiel protecteur réel de ceux-ci.

En effet, en plus de l'effet induit par leur paroi cellulaire, ces microorganismes tels que les levures et plus particulièrement *Saccharomyces boulardii* ou *Saccharomyces cerevisiae* seraient capable d'exprimer des inhibiteurs dirigés directement contre des agents enteropathogéniques tels que *Vibrio cholerae, Clostridium difficile ou Escherichia coli entéropathogénique (EPEC).* De tels inhibiteurs peuvent être des enzymes lysant les toxines ou des molécules induisant des mécanismes de défense en se liant à des récepteurs des cellules intestinales. D'autres microorganismes tels que les microalgues *Chlorella vulgaris, Chlorella saccharophila, Scenedesmus, Chlamydomonas reinhardtii* ou *Dunaliella salina* seraient capables d'exprimer des agents antioxydants, par exemple la superoxyde dismutase.

Cependant, l'art antérieur ne décrit pas un mode d'administration de ces organismes eucaryotes à paroi polysaccharidiques contenus dans le bol alimentaire ou apportés en complément qui soit tout à la fois a) d'obtention aisé c'est-à-dire sans préparation, purification ou extraction particulière, b) qui constitue un apport en agents nutritionnels directement assimilables et c) qui induise un effet protecteur pour la santé des animaux carnivores ou omnivores qui soit de façon concomitante amélioré et de spectre plus large (i) du fait des propriétés des parois cellulaires tout en ayant (ii) une libération massive d'enzymes protectrices actives ou d'agents protecteurs non dénaturés ou dégradés par un passage préalable dans l'estomac.

Afin de résoudre ces différents problèmes de l'art antérieur, l'invention porte sur une composition comprenant un ou plusieurs organismes eucaryotes à paroi polysaccharidique et une ou plusieurs maltodextrines branchées.

Un effet synergique de ce mélange a été mis en évidence (i) dans la stimulation de la flore intestinale (ii) dans la production d'enzymes par cette flore intestinale (iii) dans la protection de la santé intestinale par la libération massive d'agents actifs tels que notamment des agents antioxydants ou d'inhibiteurs d'agents pathogènes par la lyse de l'organisme eucaryote à paroi polysaccharidique.

Les organismes eucaryotes à paroi polysaccharidique seuls ne permettent pas une telle augmentation de la flore ni une telle libération de leur contenu intracellulaire. De même, les maltodextrines branchées seules ne permettent pas une telle augmentation de la flore intestinale.

L'utilisation de maltodextrines branchées associées à des organismes eucaryotes à paroi polysaccharidique permet d'avoir une amélioration du bien être intestinal en augmentant la flore d'un niveau supérieur à la simple utilisation de maltodextrines branchées ou toute autre fibre indigestible soluble seule ou en mélange. De plus, ce mélange permet tout à la fois, un apport en nutriments utilisables par la flore, entretenant et exacerbant l'induction de sa croissance, tout en apportant des agents actifs et des agents nutritionnels assimilables par l'intestin du fait même de la libération du contenu cytoplasmique de l'organisme à paroi polysaccharidique.

La demanderesse a mis en évidence l'effet synergique de ce mélange aussi bien dans la croissance de la flore que dans la lyse des organismes à paroi polysaccharidique. Ainsi, suivant l'organisme choisi, son effet sur la santé peut être ciblé. Cet effet peut être le fait d'un apport d'agents anti-oxydants tels que le sélénium, la superoxyde dismutase, les caroténoïdes, les vitamines, la chlorophylle ou les phycobiliprotéines, ou d'un apport d'agents détoxifiants, anti-inflamatoires, d'inhibiteurs de l'adhésion d'agents pathogènes (bactéries, virus, parasites). L'effet recherché peut être simplement un bien être intestinal par une augmentation de la flore, augmentation supérieure à celle observée par la simple absorption de maltodextrines branchées seules.

Le caractère particulièrement avantageux de cette composition ne réside pas dans la simple adjonction d'une maltodextrine branchée soluble à un organisme eucaryote à paroi polysaccharidique et donc dans la simple addition des effets de chacun des composés du mélange. De fait, il s'agit d'une réelle coopération entre ces deux composés du mélange, en l'obtention d'un effet synergique dans l'induction tout à la fois de la croissance de la flore et de l'expression d'enzymes glycolytiques. Cet effet est la conséquence de deux effets inattendus et consécutifs. Le premier est l'induction de l'expression d'enzymes glycolytiques dirigées contre les parois des cellules eucaryotes en quantité suffisante pour induire une première lyse des cellules. Le second effet est surprenant en ce que, par un apport massif de nutriments et de résidus de parois, les cellules lysées exacerbent à la fois la croissance de la flore et l'induction de l'expression d'enzymes glycolytiques. De façon surprenante, ces mêmes enzymes seront responsables de l'entretien de ce phénomène en induisant une lyse massive des cellules ayant conservées leur intégrité.

Absorbés seuls, les organismes eucaryotes à paroi polysaccharidique tels que par exemple les microalgues les champignons ou les végétaux sont considérés par l'art antérieur comme indigestibles pour l'homme ainsi que pour les autres animaux omnivores ou carnivores et donc pas ou peu assimilables. Ils n'entrainent donc pas seuls, une stimulation de la synthèse d'enzymes glycolytiques de la flore. Par contre, de façon surprenante, la composition selon l'invention résout ce problème et peut donc être utilisée comme complément alimentaire puisque les nutriments contenus dans les organismes eucaryotes à paroi polysaccharidique, sont, selon l'invention, libérés dans la lumière de l'intestin et deviennent donc disponibles pour être assimilés ou actifs vis-à-vis de la flore ou de la muqueuse intestinale. Une méthode pour le maintient ou l'amélioration de la santé intestinale comprenant une étape d'absorption de la composition selon l'invention est efficace notamment vis-à-vis des syndrômes entériques ou des cancers liés aux divers agressions de la flore ou de la muqueuse intestinale par des agents pathogènes, des radicaux libres ou des xénobiotiques. En effet, la croissance de la flore observée est plus importante qu'avec des compositions comprenant uniquement des maltodextrines branchées. Cette composition peut être indiquée seule ou en complément dans la prévention ou le traitement de syndromes intestinaux, de cancers intestinaux ou d'inflammations intestinales. On peut aussi envisager la prise de ce mélange afin d'améliorer la digestibilité et donc la biodisponibilité des nutriments d'origine végétale ingérés indépendamment par un individu.

Selon une variante avantageuse, la composition selon l'invention comprend des maltodextrines branchées présentant entre:
- 15 et 50 % de liaisons glucosidiques 1-6, préférentiellement entre 22 % et 45 %, plus préférentiellement entre 27 et 34 %,
- une teneur en sucres réducteurs inférieure à 20 %, préférentiellement comprise entre 2 et 20%, plus préférentiellement entre 3 et 16 %, encore plus préférentiellement entre 3 et 12 %,
- un indice de polymolécularité inférieur à 5, préférentiellement compris entre 0,5 et 4, plus préférentiellement entre 1 et 3,5 et
- une masse moléculaire moyenne en nombre Mn inférieure à 4500 g/mole préférentiellement, comprise entre 600 et 4000 g/mole plus préférentiellement comprise entre 1000 et 2700 g/mole.

Les liaisons contenues dans ces fibres seraient très proches structurellement de celles retrouvées dans les parois cellulaires des cellules eucaryotes et pourraient faciliter l'induction de la flore dans la synthèse d'enzymes dirigées contre leur parois. Les organismes eucaryotes à paroi polysaccharidique sont choisis parmi, les levures et les microalgues. L'article Bomba et al British Journal of Nutrition vol 88, no. Supplement 1, 2002-09-01 pp. S 95-S99 suggère l'amélioration de l'effet probiotique de bactéries (*lactobacacillus paracasei*) par le fait que ces bactéries utilisent pour leur développement des maltodextrines particulières, les maltodextrines KMS X-70.
Il n'est nullement fait référence dans ce document à une composition comprenant un ou plusieurs organismes eucaryotes à paroi polysaccharidique choisis parmi les levures ou les microalgues.

Le document FR2884422, au nom de la Demanderesse, divulgue l'utilisation de maltodextrines branchées toutefois il n'est pas fait mention dans ce document d'une quelconque association avec des microalgues ou levures comme dans la présente invention.

Le document WO 2006/112364A au nom de DAINIPPON concerne la culture de bactéries lactiques dans un milieu contenant une microalgue et, éventuellement des GOS.

Il n'est nullement fait mention dans ce document à une composition comprenant à la fois au moins une microalgue et au moins une maltodextrine branchée, et encore moins à une composition comprenant à la fois au moins une levure et au moins une maltodextrine branchée.

Une augmentation de la production par la flore intestinale de l'α-glucosidase, de la β glucosidase, de la β galactosidase, de l'estérase, de la cellobiohydrolase et de la β-xylosidase suite à l'ingestion de la composition selon l'invention a été mise en évidence. Ces enzymes sont des marqueurs d'une induction importante de la flore associée à une augmentation en masse de la flore signe d'une croissance concomitante de celle-ci. Ainsi, la particularité de la composition selon l'invention, est la présence de ces organismes qui servent à la fois de substrats aux enzymes produits et donc d'inducteurs de la croissance de la flore mais aussi d'inducteurs de la production d'enzymes glycolytiques en association avec les maltodextrines branchées. La composition selon l'invention permet d'induire spécifiquement des enzymes glycolytiques capables d'hydrolyser les parois des algues et des levures. Ainsi, ce large spectre d'application permet une adaptabilité de la composition selon l'invention à l'effet souhaité, à la carence à combler.

Selon la présente invention, les organismes eucaryotes à paroi polysaccharidique sont des organismes unicellulaires. Parmi les champignons, les organismes eucaryotes unicellulaires selon l'invention sont les levures. Parmi les végétaux, les organismes eucaryotes unicellulaires selon l'invention sont les microalgues. Avantageusement les microalgues sont choisies parmi les viridiplantae, les labyrinthulides, les haptophytes, les rhodophytes et les alveolates et leur combinaison préférentiellement les chlorophyta et les labyrinthulides et leur combinaison et encore plus préférentiellement *chlorella, Scenedesmus, les Dunaliella, Haematococcus, Schizochytrium, Thraustochytrium* et leur combinaison.

L'effet avantageux des microalgues est lié à leur caractère unicellulaire et à leurs conditions de culture relativement aisées. Ainsi, ces organismes sont utilisables sans traitement préalable, sans création de déchets et dans leur totalité. Par opposition aux plantes à tige, toute la matière sèche est utilisable : il n'y a pas de parties non utilisables du fait d'une caractéristique inhérente à une différenciation cellulaire, à une spécialisation d'un organe ou à une réduction d'expression d'une protéine.

Selon l'invention, la lyse des microalgues permet une libération massive de leur contenu intracellulaire non observée dans le cas de l'utilisation de la microalgue seule. Ces microalgues sont particulièrement indiquées pour leur capacité à fixer des toxines, pour leur richesse en anti-oxydants mais aussi en nutriments. Cette utilisation permet donc d'optimiser l'effet observé lors de l'absorption seule de l'organisme eucaryote à paroi polysaccharidique.

Ces microalgues sont particulièrement nutritives de par leur richesse en protéines, en acides gras polyinsaturés de type oméga 3 à longues chaînes par rapport aux végétaux terrestres.

Selon une seconde variante de l'invention, les organismes eucaryotes à paroi polysaccharidique sont des levures choisies parmi *Saccharomyces boulardii, Saccharomyces cerevisiae* et *Pichia.*

Ces levures sont particulièrement avantageuses en ce qu'elles sont particulièrement riche en protéines, en acides nucléiques et en ce qu'elles sont impliquées dans l'amélioration de la santé intestinale.

De façon préférentielle, la composition comporte un rapport en poids d'organismes eucaryotes à paroi polysaccharidique/Maltodextrines branchées allant de 5/95 à 90/10 préférentiellement de 20/80 à 80/20 et plus préférentiellement de 25/75 à 75/25.

De façon avantageuse, les maltodextrines branchées peuvent être en mélange avec des dextrines, des galacto-oligosaccharides (GOS), des fructo-oligosaccharides (FOS), des oligosaccharides oléagineux ou protéagineux, du fructane, de l'inuline, du polydextrose, des gluco-oligosaccharides, du lactosucrose, et leurs mélanges.

De façon préférentielle, la composition selon l'invention comprend en outre un composé choisi dans le groupe comprenant les dextrines, les galacto-oligosaccharides (GOS), les fructo-oligosaccharides (FOS), les oligosaccharides oléagineux ou protéagineux, le fructane, l'inuline, le polydextrose, les gluco-oligosaccharides, le lactosucrose ou leurs mélanges.

Les maltodextrines branchées plus encore que les autres fibres indigestibles solubles, exacerbent du fait de leurs liaisons atypiques et variées une induction des diverses enzymes hydrolytiques de la flore intestinale.

Parmi les oligosaccharides solubles d'origine oléagineuse ou protéagineuse, on peut citer le soja, le colza ou le pois dont les oligosaccharides solubles sont particulièrement avantageux de part la présence de liaisons oligosaccharidiques variées.

Dans l'associations maltodextrines branchées / organismes eucaryotes à paroi polysaccharidique, les organismes eucaryotes à paroi polysaccharidique préférés sont : parmi les plantes, les microalgues telles que chlorella, Schizochytrium, Thraustochytrim ou leurs mélanges ;parmi les champignons, les levures telles que *Saccharomyces boulardii, Saccharomyces cerevisiae* et *Pichia.*

A titre illustratif, on administrera de préférence de l'ordre de 2 à 100g de maltodextrines branchées pour 0,3 à 20g de organismes eucaryotes à paroi polysaccharidique par jour pour un homme, préférentiellement 5 g à 20 g de maltodextrines branchées pour 1,5 g à 6 g de organismes eucaryotes à paroi polysaccharidique par jour, pour un rapport en poids de organismes eucaryotes à paroi polysaccharidique / maltodextrines branchées allant de 5/95 à 90/10 préférentiellement de 20/80 à 80/20 et plus préférentiellement de 25/75 à 75/25.

Selon l'invention, la composition comprend 0,5 à 30 %, de préférence 5 à 15 % en poids sec de la combinaison maltodextrines branchées / organismes eucaryotes à paroi polysaccharidique

L'administration orale peut être ponctuelle, par cure de plusieurs jours ou plusieurs semaines ou être chronique.

La composition selon l'invention est de préférence sous une forme choisie parmi les formes solide, de poudre, de comprimé, d'un suppositoire, liquide, d'une émulsion ou d'un sirop.

Avantageusement la composition conforme à l'invention peut se présenter sous une forme prête à l'emploi sous forme solide telle que par exemple de poudre, de comprimé, d'un suppositoire, ou encore, sous forme liquide, d'une émulsion ou d'un sirop ou sous forme de boisson, comme un jus de fruit, une soupe, ou encore sous forme de yaourts ou incorporé dans les céréales du petit déjeuner.

Les préparations à administration orale peuvent comporter tout excipient ou véhicule usuel. Elles peuvent consister en des poudres, granulés, solutions ou autres et éventuellement, incorporer d'autres principes médicinaux ou principes actifs.

Avantageusement la composition conforme à l'invention peut comprendre en outre au moins un agent actif ou un nutriment destiné à la prévention et/ou le traitement de syndromes intestinaux tels que le syndrome du côlon irritable, la tourista, d'inflammations intestinales, de maladies Inflammatoires Chroniques de l'Intestin, de cancers intestinaux, de maladies liées à l'alimentation, la prévention de maladies liées à l'âge, la complémentation alimentaire, l'induction de la flore intestinale, l'augmentation de la résistance à l'effort, l'amélioration de la digestibilité de nutriments d'origine végétale, l'obtention d'un effet protecteur pour la santé intestinale d'un animal omnivore ou carnivore.

Selon un autre aspect de la présente invention, la composition selon la présente invention peut être utilisée comme médicament.

Avantageusement la composition selon la présente invention peut être utilisée pour la prévention et/ou le traitement de syndromes intestinaux tels que le syndrome du côlon irritable, la tourista, d'inflammations intestinales, de maladies Inflammatoires Chroniques de l'Intestin, de cancers intestinaux, de maladies liées à l'alimentation, la prévention de maladies liées à l'âge, la complémentation alimentaire, en particulier dans le cas de carences, d'un animal omnivore ou carnivore.

Selon un autre aspect de la présente invention, la composition selon la présente invention peut être utilisée de manière non thérapeutique pour l'induction de la flore intestinale, l'augmentation de la résistance à l'effort, l'amélioration de la digestibilité de nutriments d'origine végétale, l'obtention d'un effet protecteur pour la santé intestinale et la complémentation alimentaire, chez un animal sain omnivore ou carnivore.

Avantageusement, l'invention porte de plus, sur une méthode de libération contrôlée et localisée de nutriments ou d'agents actifs dans le colon d'un animal omnivore ou carnivore comportant une flore intestinale, ladite méthode comprenant une étape d'ingestion concomitante ou simultanée de maltodextrines branchées et d'organismes eucaryotes à paroi polysaccharidique contenant lesdits nutriments ou agents actifs. L'ingestion des maltodextrines branchées et des organismes eucaryotes à paroi polysaccharidique induit la lyse des parois cellulaires des organismes eucaryotes à paroi polysaccharidique dans la lumière de l'intestin de l'animal. Ainsi, cette méthode permet d'augmenter la digestibilité des organismes eucaryotes à paroi polysaccharidique, de potentialiser leurs effets sur la santé, tout en augmentant leur potentiel nutritionnel.

De manière préférentielle, les organismes eucaryotes à paroi polysaccharidique sont modifiés génétiquement ou issu d'une sélection.

Par agents actifs, on entend des agents protéiques, glycaniques, biochimiques ou des acides nucléiques ayant un effet bénéfique sur la santé tels que des enzymes ou molécules antioxydantes, des enzymes ou molécules ayant un effet anti-inflammatoire ou inhibiteur pour certains microorganismes synthétisant des principes entéropathogéniques, des molécules protectrices pour la flore intestinales ou la muqueuse intestinale.

Par ailleurs, l'invention porte également sur l'utilisation d'au moins un organisme eucaryote à paroi polysaccharidique en combinaison avec des maltodextrines branchées pour induire la croissance de la flore intestinale d'un animal omnivore ou carnivore et/ou comme complément alimentaire.

L'invention porte également sur une méthode pour le maintien et/ou amélioration de la santé d'un animal omnivore ou carnivore consistant en une première étape d'administration de maltodextrines branchées et une seconde étape concomitante ou séparée d'administration d'au moins un organisme eucaryote à paroi polysaccharidique choisi parmi les levures et les microalgues.

L'invention a également pour objet une méthode de complémentation alimentaire d'un animal omnivore ou carnivore comprenant une première étape d'administration de maltodextrines branchées et une seconde étape concomitante ou séparée d'administration d'organismes eucaryotes à paroi polysaccharidique préférentiellement choisis parmi les végétaux, les champignons et leur combinaison.

L'invention porte également sur un Kit pour le traitement thérapeutique ou prophylactique d'un animal omnivore ou carnivore comprenant :
a) une première composition selon l'invention; et
b) une deuxième composition comprenant au moins un agent actif ou un nutriment préférentiellement, ledit agent actif est destiné à l'induction de la flore intestinale, au maintien de la santé, à la complémentation alimentaire ou à la prévention de maladies liées à l'âge d'un animal omnivore ou carnivore.

Ladite composition peut être utilisée chez l'homme, mais également chez l'animal et plus particulièrement chez le chat, le chien, le cochon, le lapin ou les autres animaux qui sont sensibles à l'inflammation intestinale, des animaux présentant une diminution de leur immunité, ou des animaux dont l'activité ou la résistance à l'effort nécessite un apport en nutriments tels que les chevaux ou les chiens de course. Ladite composition peut être envisagée comme complément alimentaire pour des animaux élevés en dehors de leur milieu naturel, tels que les poissons par exemple.

Cette composition est proposée pour la complémentation alimentaire pour prévenir ou pour complémenter le traitement de maladies liées à l'alimentation ou à l'âge, de syndromes métaboliques, de Maladies Inflammatoires Chroniques de l'Intestin (ou MICI), de syndromes tels que le syndrome du côlon irritable, pour la prévention ou le traitement de personnes atteintes de tourista, de douleurs abdominales dont l'étiologie est souvent méconnue, des personnes souffrant ou sujettes à des carences alimentaires tels que des végétariens ou végétaliens voire des personnes âgées, des personnes de santé fragile ou convalescentes.

Ladite composition est enfin particulièrement adaptée aux sujets stressés dont le stress se manifeste au niveau digestif.

L'invention sera mieux comprise à la lecture des exemples qui suivent et qui se veulent illustratifs et non limitatifs.

### Exemple 1 :

L'effet de différentes fibres solubles ou insolubles sur les activités glucosidases de la flore intestinale chez le rat de laboratoire est étudié. Les fibres solubles sont des maltodextrines branchées selon l'invention, des FOS et du polydextrose, les fibres insolubles sont des fibres de cellulose.

Les maltodextrines branchées choisies dans cet exemple présentent entre 15 et 35 % de liaisons glucosidiques 1→6, une teneur en sucres réducteurs comprise entre 2 et 5 %, un indice de polymolécularité inférieur à 5 et une masse moléculaire moyenne en nombre Mn comprise entre 2000 et 3000 g/mole :

| | |
|---|---|
| Sucres réducteurs | 2,3 |
| Mn (g/mole) | 2480 |
| Mw (g/mole) | 5160 |
| Liaison 1,2 (%) | 10 |
| Liaison 1,3 (%) | 12 |

| | |
|---|---|
| Liaison 1,4 (%) | 49 |
| Liaison 1,6 (%) | 29 |

Elles présentent en outre, un taux de fibres totales de 90 % sur sec, déterminé selon la méthode AOAC (N°2001-03).

40 rats OFA d'origine Sprague Dawley sont répartis en 4 groupes qui reçoivent dans leur alimentation un régime alimentaire dont le détail figure dans le tableau 1 suivant.

Le groupe 4 reçoit une alimentation complémentée par des fructo-oligosaccharides (FOS)(RAFTILOSE® P95 commercialisé par la société ORAFTI).

Les groupes 5 et 6 reçoivent une alimentation complémentée respectivement par du polydextrose et de la cellulose.

**Tableau 1**

| Lot | Aliment et produit testé |
|---|---|
| 1 | Aliment AO4C |
| 2 | Aliment AO4C + 10 % glucose |
| 3 | Aliment AO4C + 10 % MDB |
| 4 | Aliment AO4C + 10 % FOS |
| 5 | Aliment AO4C + 10 % polydextrose |
| 6 | Aliment AO4C + 10 % cellulose |

Après une semaine d'isolement au cours de laquelle les animaux reçoivent une alimentation standard et de l'eau potable, les rats consomment la nourriture durant 36 jours.

A J₀, les animaux sont mis à jeun 24 heures. La boisson est donnée ad libitum. A J₁, les fèces sont recueillies.

Le régime décrit du tableau 2 est donné aux animaux.

A J₂₈, les animaux sont mis à jeun 24 h. La boisson est donnée ad libitum.

A J₂₉, les fèces sont à nouveau recueillies.

A J₃₆, les animaux sont sacrifiés.

Une observation macroscopique générale des organes est réalisée. Les coecum sont ligaturés et prélevés. Les cæcum pleins, les contenus cæcaux et les cæcum vides sont pesés.

Les activités enzymatiques des fèces sont évaluées également (α-glucosidase et β-glucosidase).

Le tableau 2 regroupe les activités enzymatiques des fèces déterminées respectivement à J₀ et à J₂₉.

**Tableau 2**

| | J₀ | | J₂₉ | |
|---|---|---|---|---|
| Lot | α-glucosidase (Uabs/min/g de fèces) | β-glucosidase (Uabs/min/g de fèces) | α-glucosidase (Uabs/min/g de fèces) | β-glucosidase (Uabs/min/g de fèces) |
| 1 | 3,23 ± 1,17 | 4,40 ± 2,86 | 5,62 ± 1,24 | 6,08 ± 1,39 |
| 2 | 3,19 ± 1,72 | 3,86 ± 2,03 | 5,97 ± 2,60 | 6,74 ± 3,38 |
| 3 | 3,37 ± 1,85 | 2,55 ± 1,11 | 23,09 ± 7,29 | 24,21 ± 9,10 |
| 4 | 3,10 ± 1,37 | 2,94 ± 1,19 | 15,32 ± 3,91 | 9,94 ± 3,05 |
| 5 | 3,15 ± 1,67 | 2,64 ± 1,10 | 13,22 ± 4,03 | 10,02 ± 2,94 |
| 6 | 3,22 ± 1,64 | 3,55 ± 2,10 | 6,08 ± 2,02 | 6,68 ± 2,98 |

A J₀, les activités des lots sont identiques au lot témoin 1. A J₂₉, les activités glucosidases sont très fortement augmentées par l'administration de 10 % de MDB. On observe par contre, une augmentation plus légère pour les animaux recevant 10 % de FOS ou de polydextrose. Dans le cas de la cellulose aucune augmentation significative n'est observée.

En effet, des augmentations de 310 % et de 298 % sont observées pour respectivement l'α-glucosidase et la β-glucosidase du lot recevant de la MDB par rapport au lot témoin, alors que les augmentations ne sont respectivement que de 172 et 63 % pour le lot FOS et de 135% et 64% pour le lot polydextrose.

Les MDB ont des caractéristiques beaucoup plus intéressantes que les FOS ou le polydextrose et permettent une induction de l'activité des glucosidases beaucoup plus importante. Par contre, les fibres insolubles n'ont elles, aucune incidence sur l'activité glucosidase.

### Exemple 2 :

Le métabolisme d'une microalgue, la Chlorelle et d'un champignon, la Levure a été étudié chez le rat en comparaison d'une maltodextrine branchée (MDB) et du Polydextrose (POLY) pendant 28 jours. La MDB et le polydextrose sont identiques à ceux de l'exemple précédant. En parallèle, les Chlorelles ou Levures ont été associées à la maltodextrine branchée pour étudier les effets de l'association d'un organisme eucaryote à paroi polysaccharidique et d'une fibre indigestible soluble.

Les produits testés sont introduits dans un aliment standard pour rats de laboratoire à raison d'une dose fixe de 5%, seuls ou en mélange avec un autre produit, selon le tableau 3 présenté ci-dessous.

**Tableau 3**

| N° de lot | Produits testés |
|---|---|
| Lot 1 (témoin) | - |
| Lot 2 (C) | 5% de Chlorelles |
| Lot 3 (MDB) | 5% de MDB |
| Lot 4 (Y) | 5% de Levures |
| Lot 5 (MDB+C) | 5% de MDB + 5% de Chlorelles |
| Lot 6 (MDB+Y) | 5% de MDB + 5% de Levures |
| Lot 7 (POLY) | 5% de POLY |
| Lot 8 (POLY+C) | 5% de POLY + 5% de Chlorelles |
| Lot 9 (POLY+Y) | 5% de POLY + 5% de Levures |

La Chlorelle testée est une *Chlorella Vulgaris.* La levure testée est un *Saccharomyces Cerevisiae.*

Après une semaine de quarantaine au cours de laquelle les animaux reçoivent une alimentation standard et de l'eau potable, les rats sont randomisés sur la base de leur poids et attribués à un lot d'étude.

Les rats participant à cette étude sont des rats OFA origine Sprague-Dawley, mâles. Leur poids est compris entre 100 et 125g à la réception. Ils sont hébergés par 2 dans des cages Makrolon.

En cours d'étude, différents paramètres sont évalués : observation clinique, évolution pondérale, consommation d'aliment, consommation de boisson.

A J-1 et J20, les animaux sont placés individuellement en cage à métabolisme pendant 24 heures. Pendant cette période, ils sont à jeun et reçoivent de l'eau potable à volonté.

A J0 et J21, les fèces 24 heures sont recueillies. Elles sont pesées humides et immédiatement congelées à -20°C. Elles seront ensuite lyophilisées pour une période de 48 à 72 heures, pesées sèches après lyophilisation et broyées. Les différentes analyses seront réalisées dans les 24 heures sur ces fèces broyées.

A J14, des fèces sont recueillies directement au niveau de l'anus des animaux. Une quantité minimale de 3 grammes est recueillie pour chaque animal. Ces fèces sont pesées humides puis congelées immédiatement à - 20°C en attente d'être analysées.

Sur les fèces lyophilisées (recueillies à J-1 et J20), les activités enzymatiques des α-glucosidases, β-glucosidases, β-galactosidases, estérases, cellobiohydrolases et β-xylosidases sont réalisées par méthode spectrophotométrique. Les substrats utilisés sont respectivement : p-nitrophényl-α-D-glucopyranoside, p-nitrophényl-β-D-glucopyranoside, p-nitrophényl-β-D-galactopyranoside, p-nitrophénylacétate, p-nitrophénylcellobioside, p-nitrophénylxylopyranoside. Avant de quantifier ces activités enzymatiques, les enzymes sont extraites par une succession d'étapes d'agitation, de centrifugation, de lavage. Les résultats d'activités enzymatiques sont exprimés en unité d'absorbance par minute (ou heure pour les activités cellobiohydrolases et β-xylosidases) et par gramme de fèces sèches.

Sur les fèces congelées (recueillies à J14), l'activité antioxydante est réalisée par la méthode du TEAC assay (Trolox Equivalent Antioxydante Capacity). L'objectif de ce test est de générer un radical libre (ABTS●+ de couleur bleue-verte) à partir d'un mélange d'une solution d'ABTS incolore avec du persulfate de potassium. La décoloration de l'espère radicalaire par la réaction avec les antioxydants des fèces testées permet de déterminer une capacité antioxydante globale. Cette décoloration est suivie par spectrophotométrie. Les résultats sont exprimés en pourcentage d'inhibition TEAC par rapport à un témoin négatif n'entrainant pas de décoloration.

L'analyse statistique des résultats a été réalisée par un test d'homogénéité des variances (test de Barlett) suivie d'une analyse de la variance par une ANOVA si le résultat était non significatif ou un test de Kruskall et Wallis et un test de Mann et Whitney si le résultat était significatif. Les lots ont été comparés entre eux et par rapport au lot témoin. Seules les comparaisons suivantes seront présentées :
- Lot 1 (témoin) versus tous les lots
- Lot 5 (MDB+C) versus Lot 2 (C)
- Lot 5 (MDB+C) versus Lot 3 (MDB)
- Lot 6 (MDB+Y) versus Lot 4 (Y)
- Lot 6 (MDB+Y) versus Lot 3 (MDB)
- Lot 8 (POLY+C) versus Lot 2 (C)
- Lot 8 (POLY+C) versus Lot 7 (POLY)
- Lot 9 (POLY+Y) versus Lot 4 (Y)
- Lot 9 (POLY+Y) versus Lot 7 (POLY)

Dans les tableaux, un chiffre est noté : il indique le lot vis-à-vis duquel le résultat est significatif. Les symboles T, *, **, *** indiquent le niveau de significativité, respectivement : tendance, p<0.05, p<0.01, p<0.001.

Les résultats montrent que l'évolution pondérale, la consommation d'aliment et la consommation de boisson évoluent de façon identique entre les lots. Aucune observation clinique particulière n'a été observée en cours d'étude.

Les résultats des activités enzymatiques des α-glucosidases (α-Glc), β-glucosidases (β-Glc), β-galactosidases (β-Gal), estérases, cellobiohydrolases (CBH) et β-xylosidases (β-Xyl) mesurées à J0 sont résumés dans le tableau 4 suivant :

**Tableau 4**

| Lot | α-Glc | β-Glc | β-Gal | estérases | CBH | β-Xyl |
|---|---|---|---|---|---|---|
| 1 Témoin | 6.6±1. 4 | 9.2±2.5 | 28.2±7 .8 | 121.3±33. 0 | 158.8± 78.9 | 319.9± 95.5 |
| 2 C | 7.7±1. 8 | 9.1±2.9 | 28.0±7 .7 | 121.6±33. 6 | 127.9± 53.9 | 311.6± 61. 6 |
| 3 MDB | 7.1±1. 5 | 9.2±2.8 | 26.0±7 .9 | 110.9±27. 2 | 156.7± 50.4 | 344.9± 122.1 |
| 4 | 7.5±2. | 10.6±4. | 27.7±6 | 114.4±21. | 161.6± | 360.6± |
| Y | 0 | 2 | .3 | 2 | 69.5 | 120.2 |
| 5 MDB+C | 7.1±1. 7 | 10.4±3. 1 | 26.1±8 .4 | 113.9±22. 6 | 183.3± 100.3 | 398.2± 139.6 |
| 6 MDB+Y | 6.5±0. 8 | 7.6±3.0 | 23.4±7 .9 | 103.6±28. 8 | 90.9±4 8.8 | 250.3± 93.4 |
| 7 POLY | 6.9±1. 3 | 8.7±2.1 | 25.0±6 .2 | 115.0±35. 2 | 150.0± 53.9 | 302.1± 97.7 |
| 8 POLY+C | 7.2±0. 9 | 9.4±1.3 | 27.0±7 .1 | 121±29.7 | 141.7± 50.9 | 315.7± 101.2 |
| 9 POLY+Y | 7.4±1. 5 | 9.1±2.3 | 28.0±7 .9 | 111.3±27. 9 | 125.1± 60.7 | 334.6± 115.5 |

L'analyse statistique de ces données ne montre pas de différences significatives entre les lots. En début d'étude à J0, les animaux des différents lots ont tous la même ligne de base en termes d'activités enzymatiques fécales. Les résultats des activités enzymatiques des α-glucosidases (α-Glc), β-glucosidases (β-Glc), β-galactosidases (β-Gal), estérases, cellobiohydrolases (CBH) et β-xylosidases (β-Xyl) mesurées à J21 sont résumées dans le tableau 5.

**Tableau 5**

| Lot | α-Glc | β-Glc | β-Gal | estérases | CBH | β-Xyl |
|---|---|---|---|---|---|---|
| 1 Témoin | **6.7±1.4** | **9.3±3.1** | **27.5±10.4** | **111.5±24.2** | **170.2±6 6.1** | **332.6±12 9.2** |
| *stat* | 3***-4***-5***-6***-8**-7* | 3***-4**-5***-6***-7*-8*** | 5***-6***-8*** | 5***-6***-8*** | 3**-4***-5***-6***-7**-8** | 4^{T}-5**-6**-8* |
| 2 C | **6.7±2.4** | **11.5±4.4** | **22.7±4.5** | **99.2±18.8** | **191.0±6 6.0** | **245.5±71 .3** |
| *stat* | 5***-8*** | 5***-8** | 5***-8** | 5***-8** | 5***-8** | 5***-8** |
| 3 MDB | **14.4±5. 7** | **22.1±7.2** | **28.4±10.6** | **101.6±21.8** | **544.7±2 70.3** | **391.4±98 .3** |
| *stat* | 1***-5^{T}-6** | 1***-5*-6**-7** | 5***-6***-8** | 5***-6***-8** | 1**-5*-6**-8** | 5*-6**-8* |
| 4 Y | **11.8±2. 3** | **16.3±3.8** | **31.4±8.3** | **123.0±19.4** | **327.8±7 8.5** | **435.2±12 6.5** |
| *stat* | 1***-6*** | 1**-6*** | 6*** | 6** | 1***-6*** | 1^{T}-6* |
| 5 MDB+C | **18.9±4. 8** | **31.4±7.1** | **56.3±11.3** | **162.9±26.8** | **792.6±1 69.4** | **593.2±22 6.5** |
| *stat* | 1***-2***-3^{T}-7* | 1***-2***-3*-7*-8* | 1***-2***-3***-7**-8^{T} | 1***-2***-3***-7*** | 1***-2**-3*-7* | 1**-2***-3*-7* |
| 6 MDB+Y | **34.6±14 .1** | **42.9±15. 6** | **56.4±11.5** | **170.0±26.9** | **989.3±2 08.6** | **647.4±20 8.9** |
| *stat* | 1***-3**-4***-7** | 1***-3**-4***-7* | 1***-3***-4***-7** | 1***-3***-4**-7** | 1***-3**-4***-7** | 1**-3**-4*-7** |
| 7 POLY | **9.2±2.2** | **14.7±2.7** | **28.1±7.8** | **110.9±29.7** | **321.2±1 04.7** | **353.7±57 .2** |
| *stat* | *1*-5*-6**-9** | *1*-3**-5*-6**-8^{T}-9^{T}* | *5**-6**-8** | *5***-8*-6*** | *1**-5*-6**-8** | *5*-6**-8^{T}-9^{T}* |
| 8 POLY+C | **15.2±3. 7** | **26.2±5.4** | **44.7±3.4** | **145.8±28.7** | **521.3±9 3.5** | **501.9±74 .3** |
| *stat* | *1**-2**** | *1***-2**-5*-7^{T}* | *1***-2**-3**-5^{T}-7** | *1***-2**-3**-7** | *1**-2**-3**-5*-7** | *1*-2**-3*-7^{T}* |
| 9 POLY+Y | **16.4±3. 5** | **27.7±7.2** | **43.1±4.1** | **140.7±33.3** | **601.7±1 11.1** | **500.7±54 .7** |
| *stat* | *1**-7** | *1**-4^{T}*-*6*-7^{T}* | *1***-3***-4*** | *1***-3***-4**-6** | *1**-3**-4***-6*** | *1*-3**-4*- 6*-7^{T}* |

Le tableau 6 ci-dessous représente le facteur de multiplication des activités enzymatiques des différents lots en comparaison du lot 1 (témoin). La valeur représente le ratio : (Activité enzymatique d'un lot donné)/(Activité enzymatique du lot témoin).

Un résultat supérieur à 1 montre donc que l'activité enzymatique du lot donné est supérieure à l'activité enzymatique du lot témoin.

**Tableau 6**

| Lot | α-Glc | β-Glc | β-Gal | estérases | CBH | β-Xyl |
|---|---|---|---|---|---|---|
| 2 C | 0 | 1.23 | 0.83 | 0.88 | 1.12 | 0.73 |
| 3 MDB | 2.14 | 2.37 | 1.03 | 0.91 | 3.20 | 1.17 |
| 4 Y | 1.76 | 1.75 | 1.14 | 1.10 | 1. 92 | 1.30 |
| 5 MDB+C | 2.82 | 3.37 | 2.04 | 1.46 | 4.65 | 1.78 |
| 6 MDB+Y | 5.16 | 4.61 | 2.05 | 1.52 | 5.81 | 1. 94 |
| 7 POLY | 1.37 | 1.58 | 1.02 | 0.99 | 1.89 | 1.06 |
| 8 POLY+ C | 2.27 | 2.81 | 1.62 | 1.31 | 3.06 | 1.51 |
| 9 POLY+ Y | 2.45 | 2.98 | 1.57 | 1.26 | 3.54 | 1.51 |

Le tableau 7 ci-dessous représente le facteur de multiplication des activités enzymatiques du lot « Maltodextrine branchée+Chlorelles » en comparaison des lots « Maltodextrine branchée » et « Chlorelles » ou le facteur de multiplication du lot «Polydextrose+Chlorelles » en comparaison des lots « Polydextrose » et « Chlorelles ». La valeur représente le ratio : (Activité enzymatique du lot « Produit testé+Chlorelles »)/(Activité enzymatique du lot « Produit testé») ou le ratio (Activité enzymatique du lot « Produit testé+ Chlorelles »)/(Activité enzymatique du lot « Chlorelles»).

**Tableau 7**

| Lot | α-Glc | β- Glc | β- Gal | estérases | CBH | β-Xyl |
|---|---|---|---|---|---|---|
| (MDB+C)/ C | 2.82 | 2.73 | 2.48 | 1. 64 | 4.14 | 2.41 |
| (MDB+C)/ MDB | 1.31 | 1.42 | 1.98 | 1. 60 | 1.45 | 1.51 |
| (POLY+C) /C | 2.27 | 2.28 | 1. 97 | 1.47 | 2.73 | 2.04 |
| (POLY+C) /POLY | 1.65 | 1.78 | 1.59 | 1.31 | 1. 62 | 1.42 |

Le tableau 8 ci-dessous représente le facteur de multiplication des activités enzymatiques du lot « Produit testé+Levures » en comparaison des lots « Produit testé» et « Levures». La valeur représente le ratio : (Activité enzymatique du lot «Produit testé+Levures »)/(Activité enzymatique du lot « Produit testé») ou le ratio (Activité enzymatique du lot « Produit testé+ Levures »)/(Activité enzymatique du lot « Levures»).

**Tableau 8**

| Lot | α-Glc | β-Glc | β-Gal | estérases | CBH | β-Xyl |
|---|---|---|---|---|---|---|
| (MDB+Y)/Y | 2.40 | 1. 94 | 1.98 | 1.67 | 1.81 | 1. 65 |
| (MDB+Y)/MDB | 2.93 | 2.63 | 1.79 | 1.38 | 3.01 | 1.48 |
| (POLY+Y)/Y | 1.39 | 1.70 | 1.37 | 1. 14 | 1.83 | 1.15 |
| (POLY+Y)/PO LY | 1.78 | 1.88 | 1.53 | 1.27 | 1.87 | 1.42 |

Pour les activités α-glucosidases, β-glucosidases et cellobiohydrolases, l'analyse statistique montre que les activités de tous les lots, excepté le lot « Chlorelles », augmentent significativement par rapport au lot témoin. En excluant le lot « Chlorelles », le facteur de multiplication des activités est compris entre 1.37 et 5.16 (α-glucosidases), entre 1.58 et 4.61 (β-glucosidases) et entre 1.02 et 5.81 (cellobiohydrolases) par rapport au lot témoin.

Ces activités sont statistiquement plus importantes pour le lot « Maltodextrine branchée+Chlorelles » en comparaison du lot « Maltodextrine branchée» seul ou du lot « Chlorelles » seul. Les facteurs de multiplication sont, respectivement de 1.31-2.82 pour l'α-glucosidase, 1.42-2.73 pour la β-glucosidase, 1.45-4.14 pour la cellobiohydrolase.

De même, ces activités sont statistiquement plus importantes pour le lot « Maltodextrine branchée+Levures » en comparaison du lot « Maltodextrine branchée » seul ou du lot « Levures » seul. Les facteurs de multiplication sont, respectivement de 2.40-2.93 pour l'α-glucosidase, 1.94-2.63 pour la β-glucosidase, 1.81-3.01 pour la cellobiohydrolase.

Pour les activités β-galactosidases, estérases et β-xylosidases, les lots « Maltodextrine branchée+Chlorelles » et « Maltodextrine branchée+Levures » voient leurs activités augmenter statistiquement par rapport au lot témoin. Pour ces deux lots, le facteur de multiplication des activités est de 2.04/2.05 (β-galactosidases), 1.46/1.52 (estérases) et 1.78/1.94 (β-xylosidases) par rapport au lot témoin.

Ces activités sont statistiquement plus importantes pour le lot « Maltodextrine branchée+Chlorelles » en comparaison du lot « Maltodextrine branchée » seul ou du lot « Chlorelles » seul. Les facteurs de multiplication sont, respectivement de 1.98-2.48 pour la β-galactosidase, 1.60-1.64 pour l'estérase, 1.51-2.41 pour la β-xylosidase.

De même, ces activités sont statistiquement plus importantes pour le lot « Maltodextrine branchée+Levures » en comparaison du lot « Maltodextrine branchée » seul ou du lot « Levures » seul. Les facteurs de multiplication sont, respectivement de 1.98-1.79 pour la β-galactosidase, 1.67-1.38 pour l'estérase, 1.65-1.48 pour la β-xylosidase. L'ensemble des résultats est moins marqué pour le polydextrose testé seul ou en mélange avec la Chlorelle ou les levures.

Afin de prouver l'effet synergique des produits et non l'effet cumulatif, le tableau 9 ci-dessous représente la différence d'activité enzymatique calculée entre un lot donné et le lot témoin. Sur ce même tableau, les « résultats théoriques » ont été calculés :
- l'activité théorique du lot 5 représente la somme de l'activité obtenue pour le lot 2 additionnée de l'activité du lot 3 ;
- l'activité théorique du lot 6 représente la somme de l'activité obtenue pour le lot 3 additionnée de l'activité du lot 4.
- l'activité théorique du lot 8 représente la somme de l'activité obtenue pour le lot 2 additionnée de l'activité du lot 7 ;
- l'activité théorique du lot 9 représente la somme de l'activité obtenue pour le lot 7 additionnée de l'activité du lot 4.

Le tableau 9 montre clairement que quelque soit l'activité enzymatique mesurée, les activités obtenues pour les produits testés en mélange sont très largement supérieures aux activités enzymatiques théoriques calculées. Les effets sont donc des effets synergiques et non des effets additifs.

**Tableau 9**

| | α-Glc | β-Glc | β-Gal | estérases | CBH | β-Xyl |
|---|---|---|---|---|---|---|
| 2 | 0 | 2.2 | -4.8 | -12.3 | 20.8 | -87.1 |
| C | | | | | | |
| 3 | 7.7 | 12.8 | 0.9 | -9.9 | 374.5 | 58.8 |
| MDB | | | | | | |
| 4 | 5.1 | 7.0 | 3.9 | 11.5 | 157.5 | 102.6 |
| Y | | | | | | |
| 5 | 12.2 | 22.1 | 28.8 | 51.4 | 622.4 | 260. E |
| MDB+C | | | | | | |
| 5 théorique | 7.7 | 15.0 | -3.9 | -22.2 | 395.3 | -28.3 |
| MDB+C | | | | | | |
| 6 | 27.9 | 33.6 | 28.9 | 58.5 | 819.1 | 314.8 |
| MDB+Y | | | | | | |
| 6 théorique | 12.8 | 19.8 | 4.8 | 1.6 | 532.1 | 161.4 |
| MDB+Y | | | | | | |
| 7 | 2.5 | 5.4 | 1.0 | -0.6 | 151.0 | 21.1 |
| POLY | | | | | | |
| 8 | 8.5 | 16.9 | 17.2 | 34.3 | 351.1 | 169.3 |
| POLY+C | | | | | | |
| 8 | 2.5 | 7.6 | -3.8 | -12.9 | 171.8 | -66.0 |
| théorique | | | | | | |
| POLY+C | | | | | | |
| 9 | 9.7 | 18.4 | 15.6 | 29.2 | 431.5 | 168.1 |
| POLY+Y | | | | | | |
| 9 | 7.6 | 12.4 | 4.9 | 10.9 | 308.5 | 123.7 |
| théorique | | | | | | |
| POLY+Y | | | | | | |

Afin de mettre en évidence la lyse cellulaire des microorganismes, une analyse d'un marqueur intracellulaire a été effectuée. Par opposition à la levure, la Chlorelle est décrite comme contenant de nombreux agents antioxydants telles que la chlorophylle et les vitamines par exemple. L'étude de ce marqueur spécifique de la lyse de la chlorelle dans les fèces aussi bien des rats ayant ingérés de la chlorelle (lot 2 et 5) que des levures (lot 4 et 6), permet de distinguer la lyse de la chlorelle de tout autre effet artéfactuel et non spécifique à l'organisme eucaryote, chlorelle ou levure, associé à la fibre.

Les résultats des activités antioxydantes des fèces mesurées à J14 sont résumés dans le tableau 10.

**Tableau 10**

| Lot | % d'inhibition |
|---|---|
| 1 | **409.7±28.6** |
| Témoin | |
| *stat* | 5*** |
| 2 | **413.9±37.6** |
| C | |
| *stat* | 5*** |
| 3 | **426.1±43.4** |
| MDB | |
| *stat* | 5** |
| 4 | **380.6±23.6** |
| Y | |
| *stat* | - |
| 5 | **497.0±51.9** |
| MDB+C | |
| *stat* | 1***-2***-3** |
| 6 | **419.5±30.5** |
| MDB+Y | |
| *stat* | - |
| *7* | **422.4±37.9** |
| *POLY* | |
| *stat* | - |
| *8* | **451.7±52.2** |
| *POLY+C* | |
| *stat* | - |
| *9* | **430.9±32.1** |
| *POLY+Y* | |
| *stat* | - |

La capacité antioxydante des fèces des animaux du lot « Maltodextrine branchée+Chlorelles » est statistiquement augmentée par rapport au lot témoin, par rapport au lot « Maltodextrine branchée » et par rapport au lot « Chlorelles ».

Les facteurs de multiplication pour le lot « Maltodextrine branchée+Chlorelles » sont de :
- 1.21 par rapport au lot témoin
- 1.20 par rapport au lot « Chlorelles »
- 1.16 par rapport au lot « Maltodextrine branchée ». Les résultats obtenus pour le polydextrose sont nettement moins accentués.

Afin de prouver l'effet synergique des produits et non l'effet cumulatif, le tableau 11 ci-dessous représente la différence de capacité antioxydante calculée entre un lot donné et le lot témoin. Sur ce même tableau, les « résultats théoriques » ont été calculés :
- l'activité théorique du lot 5 représente la somme de la capacité antioxydante obtenue pour le lot 2 additionnée de la capacité antioxydante du lot 3 ;
- l'activité théorique du lot 6 représente la somme de la capacité antioxydante obtenue pour le lot 3 additionnée de la capacité antioxydante du lot 4.

**Tableau 11**

| | | % d'inhibition |
|---|---|---|
| 2 | C | 4.2 |
| 3 | MDB | 16.4 |
| 4 | Y | -29.1 |
| 5 | MDB+C | 87.3 |
| 5 théorique | MDB+C | 20.6 |
| 6 | MDB+Y | 9.8 |
| 6 théorique | MDB+Y | -12.7 |
| 7 | POLY | 12.7 |
| 8 | POLY+C | 42.0 |
| 8 théorique | POLY+C | 16.9 |
| 9 | POLY+Y | 21.2 |
| 9 théorique | POLY+Y | -16.4 |

Ce tableau montre clairement que la capacité antioxydante obtenue pour le mélange maltodextrine branchée et chlorelles est très largement supérieures à la capacité antioxydante théorique calculée. Les effets sont donc des effets synergiques et non des effets additifs. Cette observation n'est pas valable pour le lot MDB+Y.

Ces résultats montrent bien que l'hydrolyse de la paroi de la Chlorelle permet la libération des antioxydants alors que cela n'est pas observé pour les animaux consommant la Chlorelle seule.

Afin de conforter les observations effectuées sur les MDB, d'autres MDB ont été testées (tableau 12) et des résultats similaires ont été obtenus.

**Tableau 12**

| | MDB1 | MDB2 | MDB3 |
|---|---|---|---|
| Mn (g/mole) | 1189 | 1232 | 2504 |
| Mw (g/mole) | 3996 | 4004 | 4602 |
| Mn/Mw | 3,4 | 3,2 | 1,8 |
| Liaison 1-6 | 33 | 32 | 31-35 |
| Sucres réducteurs | 10,4 | 9,6 | 4,1 |

Ces résultats sont extrêmement intéressants puisqu'ils démontrent un effet synergique, et non additif, entre la Maltodextrine branchée et les Chlorelles ou Levures. Dans le cas du polydextrose, on observe de la même façon un effet synergique entre le polydextrose et les Chlorelles ou levures. Cependant, le polydextrose permettant une activation des glucosidases moins importante que les maltodextrines branchées, l'effet global observé est moins intense. Ainsi, la demanderesse a montré par l'étude des activités enzymatiques dans les fèces des rats, un effet synergique du mélange de fibres solubles indigestibles et d'organismes eucaryotes à paroi polysaccharidique sur la croissance de la flore intestinale. La demanderesse a de plus montré, par l'étude des activités antioxydantes des fèces, un effet synergique du mélange de fibres solubles indigestibles et d'organisme eucaryotes à paroi polysaccharidique sur la lyse des organismes à paroi polysaccharidique. Selon toute vraisemblance, les bactéries de la flore intestinale, suite à l'induction du fait de l'ingestion du mélange sécrèteraient des enzymes capables d'hydrolyser la paroi des Chlorelles et des Levures libérant divers composés notamment azotés favorisant la croissance d'autres bactéries qui vont elles-mêmes produire des enzymes.

Salyers AA, Palmer JK, Wilkins TD.Laminarinase (beta-glucanase) activity in Bacteroides from the human colon.Appl Environ Microbiol. 1977 May;33(5):1118-24.

Robert C, Chassard C, Lawson PA, Bernalier-Donadille A. Bacteroides cellulosilyticus sp. nov., a cellulolytic bacterium from the human gut microbial community. Int J Syst Evol Microbiol. 2007 Jul;57(Pt 7):1516-20.

Kopecný J, Hajer J, Mrázek J.Detection of cellulolytic bacteria from the human colon.Folia Microbiol (Praha). 2004;49(2):175-7.

Marteau P, Pochart P, Flourié B, Pellier P, Santos L, Desjeux JF, Rambaud JC. Effect of chronic ingestion of a fermented dairy product containing Lactobacillus acidophilus and Bifidobacterium bifidum on metabolic activities of the colonic flora in humans. Am J Clin Nutr. 1990 Oct;52(4):685-8.

## Revendications

1. Composition **caractérisée en ce qu'**elle comprend :
- un ou plusieurs organismes eucaryotes à paroi polysaccharidique choisis parmi les levures et les microalgues et
- une ou plusieurs maltodextrines branchées.

2. Composition selon la revendication 1 **caractérisée en ce que** les maltodextrines branchées présentent entre:
- 15 et 50 % de liaisons glucosidiques 1-6, préférentiellement entre 22 % et 45 %, plus préférentiellement entre 27 et 34 %,
- une teneur en sucres réducteurs inférieure à 20 %, préférentiellement comprise entre 2 et 20%, plus préférentiellement entre 3 et 16 %, encore plus préférentiellement entre 3 et 12 %,
- un indice de polymolécularité inférieur à 5, préférentiellement compris entre 0,5 et 4, plus préférentiellement entre 1 et 3,5 et
- une masse moléculaire moyenne en nombre Mn inférieure à 4500 g/mole préférentiellement, comprise entre 600 et 4000 g/mole plus préférentiellement comprise entre 1000 et 2700 g/mole.

3. Composition selon l'une ou l'autre des revendications 1 et 2 **caractérisée en ce que** les organismes eucaryotes à paroi polysaccharidique sont des organismes unicellulaires.

4. Composition selon la revendication 3 **caractérisée en ce que** les organismes eucaryotes unicellulaires à paroi polysaccharidique sont des microalgues choisies parmi les *viridiplantae,* les *labyrinthulides,* les *haptophytes,* les *rhodophytes* et les *alveolates* ou leurs combinaisons préférentiellement les *chlorophyta,* les *labyrinthulides* et leur mélange.

5. Composition selon la revendication 4 **caractérisée en ce que** les organismes eucaryotes unicellulaires à paroi polysaccharidique sont des levures *choisies parmi Saccharomyces boulardii, Saccharomyces cerevisiae* et *Pichia.*

6. Composition selon l'une quelconque des revendications 1 à 5 **caractérisée en ce qu'**elle comporte un rapport en poids organismes eucaryotes à paroi polysaccharidique / maltodextrines branchées allant de 5/95 à 90/10.

7. Composition selon l'une quelconque des revendications 1 à 6 **caractérisée en ce que** la composition comprend en outre un composé choisi dans le groupe comprenant les dextrines, les galacto-oligosaccharides (GOS), les fructo-oligosaccharides (FOS), les oligosaccharides oléagineux ou protéagineux, le fructane, l'inuline, le polydextrose, les gluco-oligosaccharides, le lactosucrose ou leurs mélanges.

8. Composition selon l'une quelconque des revendications 1 à 7 **caractérisée en ce qu'**elle est sous une forme choisie parmi les formes solide, de poudre, de comprimé, d'un suppositoire, liquide, d'une émulsion ou d'un sirop.

9. Composition selon l'une quelconque des revendications 1 à 8 **caractérisée en ce qu'**elle comprend en outre au moins un agent actif ou un nutriment destiné à la prévention et/ou le traitement de syndromes intestinaux tels que le syndrome du côlon irritable, la tourista, d'inflammations intestinales, de maladies Inflammatoires Chroniques de l'Intestin, de cancers intestinaux, de maladies liées à l'alimentation, la prévention de maladies liées à l'âge, la complémentation alimentaire, l'induction de la flore intestinale, l'augmentation de la résistance à l'effort, l'amélioration de la digestibilité de nutriments d'origine végétale, l'obtention d'un effet protecteur pour la santé intestinale d'un animal omnivore ou carnivore.

10. Composition selon l'une quelconque des revendications 1 à 9, pour utilisation comme médicament.

11. Composition selon l'une quelconque des revendications 1 à 9, pour utilisation dans la prévention et/ou le traitement de syndromes intestinaux tels que le syndrome du côlon irritable, la tourista, d'inflammations intestinales, de maladies Inflammatoires Chroniques de l'Intestin, de cancers intestinaux, de maladies liées à l'alimentation, la prévention de maladies liées à l'âge, la complémentation alimentaire, en particulier dans le cas de carences, d'un animal omnivore ou carnivore.

12. Utilisation non thérapeutique de la composition selon l'une quelconque des revendications 1 à 9, pour l'induction de la flore intestinale, l'augmentation de la résistance à l'effort, l'amélioration de la digestibilité de nutriments d'origine végétale, l'obtention d'un effet protecteur pour la santé intestinale et la complémentation alimentaire, chez un animal sain omnivore ou carnivore.

## Patentansprüche

1. Zusammensetzung, welche **dadurch gekennzeichnet ist, dass** sie umfasst:
- eine oder mehrere eukaryotische Organismen mit Polysaccharid-Wand, gewählt aus Hefen und Mikroalgen, und
- ein oder mehrere verzweigte Maltodextrine.

2. Zusammensetzung nach Anspruch 1, **dadurch gekennzeichnet, dass** die verzweigten Maltodextrine zwischen:
- 15 und 50% 1-6 glykosidische Bindungen, vorzugsweise zwischen 22% und 45%, stärker bevorzugt zwischen 27% und 34%, aufweisen,
- einen Gehalt an reduzierenden Zuckern unter 20% aufweisen, vorzugsweise zwischen 2 und 20%, stärker bevorzugt zwischen 3 und 16%, noch stärker bevorzugt zwischen 3 und 12%,
- einen Polymolekularitätsindex unter 5 aufweisen, bevorzugt zwischen 0,5 und 4, stärker bevorzugt zwischen 1 und 3,5 und
- ein zahlengemitteltes Molekulargewicht Mn von weniger als 4500 g/Mol aufweisen, bevorzugt zwischen 600 und 4000 g/Mol, stärker bevorzugt zwischen 1000 und 2700 g/Mol.

3. Zusammensetzung nach dem einen oder dem anderen der Ansprüche 1 und 2, **dadurch gekennzeichnet, dass** die eukaryotischen Organismen mit Polysaccharid-Wand einzellige Organismen sind.

4. Zusammensetzung nach Anspruch 3, **dadurch gekennzeichnet, dass** die eukaryotischen einzelligen Organismen mit Polysaccharid-Wand Mikroalgen sind, gewählt aus *viridiplantae, labyrinthulides, haptophytes, rhodophytes* und *alveolates* oder deren Zusammensetzungen, vorzugsweise *chlorophyta, labyrinthulides* und deren Mischung.

5. Zusammensetzung nach Anspruch 4, **dadurch gekennzeichnet, dass** die eukaryotischen einzelligen Organismen mit Polysaccharid-Wand Hefen sind, gewählt aus *Saccharomyces boulardii, Saccharomyces cerevisiae* und *Pichia.*

6. Zusammensetzung nach einem der Ansprüche 1 bis 5, **dadurch gekennzeichnet, dass** sie ein Gewichtsverhältnis von eukaryotischen Organismen mit Polysaccharid-Wand / verzweigten Maltodextrinen aufweist, das von 5/95 bis 90/10 reicht.

7. Zusammensetzung nach einem der Ansprüche 1 bis 6, **dadurch gekennzeichnet, dass** die Zusammensetzung ferner eine Verbindung umfasst, welche gewählt ist aus der Gruppe, umfassend Dextrine, Galacto-Oligosaccharide (GOS), Frukto-Oligosaccharide (FOS), ölhaltige oder eiweißhaltige Oligosaccharide, Fruktan, Inulin, Polydextrose, Gluko-Oligosaccharide, Lactosucrose oder deren Mischungen.

8. Zusammensetzung nach einem der Ansprüche 1 bis 6, **dadurch gekennzeichnet, dass** sie in einer Form vorliegt, gewählt aus festen Formen, Pulver, Tablette, Zäpfchen, Flüssigkeit, Emulsion oder Saft.

9. Zusammensetzung nach einem der Ansprüche 1 bis 8, **dadurch gekennzeichnet, dass** diese ferner wenigstens einen Wirkstoff oder einen Nährstoff umfasst, der der Prävention und/oder der Behandlung von Darmproblemen, wie beispielsweise Reizdarm, Reisediarrhöe, Darmentzündungen, chronisch-entzündliche Darmerkrankungen, Darmkrebs, nahrungsbedingte Erkrankungen, der Prävention von altersbedingten Erkrankungen, der Nahrungsergänzung, dem Aufbau der Darmflora, der Verbesserung der Abwehrkräfte, der Verbesserung der Verdaulichkeit von Nährstoffen pflanzlicher Herkunft, oder einem Schutzeffekt für die Darmgesundheit eines tierischen Allesfressers oder Fleischfressers dient.

10. Zusammensetzung nach einem der Ansprüche 1 bis 9 zur Anwendung als Arzneimittel.

11. Zusammensetzung nach einem der Ansprüche 1 bis 9 zur Verwendung zur Prävention und/oder Behandlung von Darmproblemen, wie beispielsweise Reizdarm, Reisediarrhöe, Darmentzündungen, chronisch-entzündliche Darmerkrankungen, Darmkrebs, nahrungsbedingte Erkrankungen, zur Prävention von altersbedingten Erkrankungen, zur Nahrungsergänzung, insbesondere bei Mangelerscheinungen, bei einem tierischen Allesfresser oder Fleischfresser.

12. Nichttherapeutische Verwendung der Zusammensetzung nach einem der Ansprüche 1 bis 9 zum Aufbau der Darmflora, zur Verbesserung der Abwehrkräfte, zur Verbesserung der Verdaulichkeit von Nährstoffen pflanzlicher Herkunft, für einen Schutzeffekt für die Darmgesundheit und zur Nahrungsergänzung bei einem gesunden tierischen Allesfresser oder Fleischfresser.

## Claims

1. A composition **characterized in that** it comprises:
- one or more eukaryotic organisms with a polysaccharide wall chosen from the group of yeasts and microalgae and
- one or more branched maltodextrins.

2. The composition as claimed in claim 1, **characterized in that** the branched maltodextrins have:
- between 15% and 50% of 1,6-glucosidic linkages, preferably between 22% and 45%, more preferably between 27% and 34%,
- a reducing sugar content of less than 20%, preferably between 2% and 20%, more preferably between 3% and 16%, even more preferably between 3% and 12%,
- a polydispersity index of less than 5, preferably between 0.5 and 4, more preferably between 1 and 3.5,
and
- a number-average molecular mass Mn of less than 4500 g/mol, preferably between 600 and 4000 g/mol, more preferably between 1000 and 2700 g/mol.

3. The composition as claimed in either claim 1 or 2, **characterized in that** the eukaryotic organisms with a polysaccharide wall are unicellular organisms.

4. The composition as claimed in claim 3, **characterized in that** the unicellular eukaryotic organisms with a polysaccharide wall are microalgae chosen from the *viridiplantae,* the *labyrinthulids,* the *haptophytes,* the *rhodophytes* and the *alveolates,* or combinations thereof, preferably the *chlorophyta,* the *labyrinthulids* and the mixture thereof.

5. The composition as claimed in claim 4, **characterized in that** the unicellular eukaryotic organisms with a polysaccharide wall are yeasts chosen from *Saccharomyces boulardii, Saccharomyces cerevisiae* and *Pichia.*

6. The composition as claimed in any one of claims 1 to 5, **characterized in that** it comprises a ratio by weight of eukaryotic organisms with a polysaccharide wall/branched maltodextrins ranging from 5/95 to 90/10.

7. The composition as claimed in any one of claims 1 to 6, **characterized in that** the composition also comprises a compound chosen from the group comprising dextrins, galactooligosaccharides (GOSs), fructooligosaccharides (FOSs), oleaginous or proteaginous oligosaccharides, fructan, inulin, polydextrose, glucooligosaccharides, lactosucrose or mixtures thereof.

8. The composition as claimed in any one of claims 1 to 7, **characterized in that** it is in a form chosen from solid forms, in the form of a powder, a tablet or a suppository, or liquid forms, in the form of an emulsion or a syrup.

9. The composition as claimed in any one of claims 1 to 8, **characterized in that** it also comprises at least one active agent or one nutrient intended for the prevention and/or treatment of intestinal syndromes such as irritable bowel syndrome, or traveler's diarrhea, of intestinal inflammations, of chronic inflammatory bowel diseases, of intestinal cancers or of diet-related diseases, the prevention of age-related diseases, food supplementation, induction of the intestinal flora, increasing the resistance to physical exertion, improving the digestibility of nutrients of plant origin, and obtaining a protective effect on the intestinal health of an omnivorous or carnivorous animal.

10. The composition as claimed in any one of claims 1 to 9, as its use as a medicament.

11. The composition as claimed in any one of claims 1 to 9, for its use in the prevention and/or treatment of intestinal syndromes such as irritable bowel syndrome or traveler's diarrhea, of intestinal inflammations, of chronic inflammatory bowel diseases, of intestinal cancers, of diet-related diseases, the prevention of age-related diseases, and food supplementation, in particular in the case of deficiencies, of an omnivorous or carnivorous animal.

12. The nontherapeutic use of the composition as claimed in any one of claims 1 to 9, for inducing the intestinal flora, increasing the resistance to physical exertion, improving the digestibility of nutrients of plant origin, obtaining a protective effect on intestinal health, and food supplementation, in a healthy omnivorous or carnivorous animal.
